(19)

Europäisches
Patentamt
European
Patent Office
Office européen
des brevets

(11) **EP 2 256 496 A2**

(12) **EUROPEAN PATENT APPLICATION**

(43) Date of publication:
**01.12.2010 Bulletin 2010/48**

(21) Application number: **10007962.3**

(22) Date of filing: **20.09.2007**

(51) Int Cl.:
*G01N 33/50* (2006.01)          *G01N 33/68* (2006.01)
*G01N 33/74* (2006.01)

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **21.09.2006   US 846308 P**
**18.12.2006   US 875696 P**

(62) Document number(s) of the earlier application(s) in accordance with Art. 76 EPC:
**07826471.0 / 2 064 545**

(71) Applicant: **Piramal Life Sciences Limited**
**Mumbai 400 013**
**MAH (IN)**

(72) Inventors:
• **Marita, Rosalind Adaikalasamy**
  **Mumbai 400 063 (IN)**
• **Sharma, Somesh**
  **Mumbai 400 063 (IN)**
• **Anthony, Jessy**
  **Mumbai 400 063 (IN)**

• **Kelkar, Aditya**
  **Mumbai 400 063 (IN)**
• **Bhumra, Sujit Kaur**
  **Mumbai 400 063 (IN)**
• **Ghate, Aditee**
  **Mumbai 400 063 (IN)**
• **Nemmani, Kumar Venkata Subrahmanya**
  **Mumbai 400 063 (IN)**
• **Deka, Nabajyoti**
  **Mumbai 400 063 (IN)**
• **Gangopadhyay, Ashok Kumar**
  **Mumbai 400 063 (IN)**

(74) Representative: **Richards, Michèle E. et al**
**Fry Heath & Spence LLP**
**The Gables**
**Massetts Road**
**Horley**
**Surrey RH6 7DQ (GB)**

Remarks:
This application was filed on 30-07-2010 as a divisional application to the application mentioned under INID code 62.

(54)     **Method for identifying compounds that act as insulin-sensitizers**

(57)     The present invention relates to a method for identifying compounds that act as insulin-sensitizers. The method can include screening of test compounds in two assays of insulin sensitivity. This method can identify lead compounds for the treatment of disorders caused by insulin resistance to glucose uptake. This invention also includes methods for treating insulin resistance and related disorders.

The present invention further relates to compounds for use in the treatment of disorders caused by insulin resistance to glucose uptake and disorders related thereto.

**EP 2 256 496 A2**

**Description**

**CROSS-REFERENCE TO RELATED APPLICATIONS**

This application is related to our copending PCT applications entitled: COMPOUNDS FOR THE TREATMENT OF METABOLIC DISORDERS (PCT/IB2007/053811) and COMPOUNDS FOR THE TREATMENT OF METABOLIC DISORDERS (PCT/IB2007/053812) filed on the same date as the present application.

**FIELD OF INVENTION**

The present invention relates to a method for identifying compounds which act as insulin-sensitizers. The method can include screening of test compounds in two assays of insulin sensitivity. This method can identify lead compounds for the treatment of diseases caused by insulin resistance. This invention also includes methods for treating insulin resistance and related disorders such as diabetes, obesity and dyslipidemia.

**BACKGROUND OF INVENTION**

Diabetes is a metabolic disorder that affects the ability to produce or use insulin in an individual. Blood glucose levels are higher than normal for individuals with diabetes. There are two main types of diabetes -Type 1 and Type 2.

In Type 1 diabetes, the pancreas does not produce insulin. Type 1 diabetes is generally diagnosed in childhood and hence, known as juvenile diabetes. This type accounts for about 5% of people with diabetes.

In Type 2 diabetes, there are two defects: i) pancreas does not produce enough insulin; ii) the tissues are unable to use insulin properly, and the resulting condition is called as insulin resistance. Type 2 diabetes is a chronic metabolic disease characterized by insulin resistance, hyperglycemia and hyperinsulinema. It represents about 95 % of the human population with diabetes. Type 2 diabetes is also commonly called "adult-onset diabetes", since it is diagnosed later in life, generally after the age of 45. In recent years Type 2 diabetes has been diagnosed in younger people, including children, more frequently than in the past.

Type 2 diabetes is a metabolic disorder characterized by elevated levels of fasting blood glucose in the affected individuals. Uncontrolled diabetes is the leading cause of blindness, renal failure, non-traumatic limb amputation and premature cardiovascular mortality. Current estimates indicate that the total annual cost of treatment of diabetes is more than $130 million in the United States alone.

The incidence of Type 2 diabetes is rapidly increasing in all parts of the world. It has been estimated that about 300 million people will be suffering from this disease by the end of this decade. The main force driving this alarming rise is the increasing prevalence of obesity among the population. Both obesity and Type 2 diabetes are characterized by peripheral tissue insulin resistance.

In normal individuals, sugars are absorbed from dietary carbohydrate sources. These are transported to the liver and peripheral tissues for utilization and storage. Insulin and its signaling system drive the central and peripheral pathways by which nutrients such as glucose are ingested, distributed, metabolized and stored. A majority of these pathways are evolutionarily conserved from worms to human. Therefore optimal insulin function is necessary for the growth and maintenance of all living organisms. Accordingly, abnormalities in insulin signaling, termed "insulin resistance", result in complications affecting the whole body in addition to causing hyperglycemia of diabetes.

Resistance to insulin-mediated glucose uptake is the fundamental abnormality in Type 2 diabetes. Various epidemiological studies have found close association between hyperinsulinaemia (a surrogate marker of insulin resistance) and cardiovascular risk factors such as hypertension, dyslipidemia, impaired glucose tolerance and obesity (Diabetes Care, 15, 318-368, 1997; Diabetes, 37, 1595-1607, 1988). The constellation of the above cardiovascular risk factors occurring in an individual has been variously referred to as metabolic syndrome or syndrome X, by clinical specialists. It is believed that metabolic syndrome affects millions of people worldwide who will eventually develop cardiovascular disease which is the main cause of death.

The pathogenesis of diabetes is not understood in great detail but it is believed that insulin resistance in skeletal muscle and fat tissue occurs very early in individuals much before the onset of hyperglycemia. Peripheral tissue insulin resistance leads to compensatory responses including increase in insulin release by the pancreatic β cells and elevated glucose production by the liver. (Diabetes, 53, 1633-42, 2004). In non-diabetic subjects with a family history of Type 2 diabetes, insulin resistance in skeletal muscle occurs before the development of diabetes (Diabetes, 41, 598-604, 1992; J. Clin. Invest., 89, 782-788, 1992). Therefore, defects in the insulin signaling pathway will not only give rise to elevated blood glucose levels but also lead to long term complications by affecting other organs such as pancreas, liver, heart, brain and vascular endothelium through hyperglycemia induced changes.

Diabetic patients either lack sufficient endogenous secretion of insulin hormone (Type 1 diabetes) or have an insulin receptor-mediated signaling pathway that is resistant to endogenous or exogenous insulin (Type 2 diabetes). In Type 2 diabetic patients, major insulin-responsive tissues such as liver, skeletal muscle and fat exhibit insulin resistance. The cause of resistance to insulin in Type 2 diabetes is complex and likely to be multi-factorial. It appears to be caused by an impaired signal from the insulin receptor to the glucose transport system and to glycogen synthase. Impairment of the insulin receptor kinase has been implicated in the pathogenesis of this signaling defect. Insulin resistance is also found in many non-diabetic individuals and may be an underlying etiologic factor in the development of the disease.

Obesity is a disorder characterized by the accumulation of excess fat in the body. Increased incidence of obesity leads to complications such as hypertension, Type 2 diabetes, atherosclerosis, dyslipidemia, osteoarthritis and certain forms of cancer.

Obesity is commonly identified by increased body weight and body mass index (BMI). People with excess body weight are characterized by peripheral tissue insulin resistance. The term 'insulin resistance' refers to decreased biological response to insulin. In obese individuals insulin resistance is often compensated by an increased secretion of insulin from the pancreas. Obese subjects exhibit hyperinsulinemia, an indirect evidence of peripheral insulin resistance. However, the body can increase insulin secretion only to a certain level. Hence if the insulin resistance continues to worsen in an obese person, eventually the body will no longer be able to compensate by stimulating insulin secretion any further. At this time, the plasma insulin levels tend to fall which in turn leads to rise in glucose levels thus precipitating

Type 2 Diabetes. Clearly, this gradual decline in insulin secretion caused by insulin resistance, initiated by excess fat accumulation, is undesirable for an individual. Hence, drugs that prevent excess fat accumulation and obesity are desirable. Thus methods and procedures to identify compounds that halt the development of insulin resistance are useful in treating obese individuals. These individuals by virtue of having pharmacological control on insulin resistance will benefit from such treatments in having fewer incidences of heart disease such as elevated blood pressure, abnormal lipid profiles and atherosclerosis.

Diabetic patients are at an increased risk of developing cardiovascular disease events due to risk factors such as dyslipidemia, obesity, hypertension and glucose intolerance. The presence of the above risk factors in an individual is collectively called metabolic syndrome. According to National Cholesterol Expert Panel's ATP III criteria, dyslipidemia is defined as a state in which an individual exhibits a combination of triglyceride levels of 150mg/dl and above, and HDL cholesterol levels of less than 40 mg/dl in men and less than 50 mg/dl in women (J. Am. Med. Association, 285, 2486-2497, 2001).

Traditional therapies of Type 2 diabetes have been aimed at reducing the hyperglycemia of diabetic patients. These include: i) compounds which increase insulin secretion from the pancreas, examples are sulfonylureas such as glibenclamide, nateglinide and repaglinide, ii) biguanides such as metformin, which act to reduce hepatic glucose production, iii) $\alpha$-glucosidase inhibitors which interfere with glucose absorption in the intestine, and lastly, iv) insulin which acts on insulin signaling pathways to reduce blood glucose. These treatments have limited efficacy and tolerability and induce side effects such as hypoglycemia and gastro intestinal (GI) disturbances.

In the recent past, a new class of drugs exemplified by pioglitazone and rosiglitazone, which act by reducing peripheral insulin resistance, has been developed. These drugs are ligands for the nuclear receptor, peroxisome proliferator-activated receptor gamma isoform (PPAR gamma), expressed primarily in the adipose tissue. Although these drugs act as insulin sensitizers in reducing blood sugar and hyperinsulinemia, this thiazolidinedione (TZD) class of drugs has limited patient compliance. The most common side effects of these PPAR gamma agonists are weight gain and fluid retention characterized by edema in the feet. In view of this, it is required to identify suitable compounds which act as insulin-sensitizers.

One of the drugs that is used as an antiobesity agent is sibutramine which acts through the central nervous system and is a serotonin reuptake inhibitor. Rise in blood pressure and increase in heart beat have been reported as side effects of this drug. Another antiobesity agent is orlistat which inhibits fat absorption in the intestine. The drug is reported to cause GI disturbances.

There remains a need to develop a method to identify compounds that can be effectively used therapeutically to treat diseases or disorders caused by insulin resistance.

## SUMMARY OF INVENTION

The present invention includes a method of identifying compounds that act as insulin sensitizers. In this method the compounds can be screened in two assays of insulin sensitivity. The method can employ a first and a second screen. The first screen can include screening the test compounds in a phenotype-based assay. The second screen can include testing compounds identified as active in the phenotype-based assay in an insulin resistance assay. This method can identify lead compounds for the treatment of diseases caused by insulin resistance to glucose uptake.

Compounds identified by the present method can be employed for treating disorders caused by insulin resistance. Accordingly, the present invention also includes a method for treating diseases caused by insulin resistance to glucose uptake and related disorders. The present invention also includes the compounds identified by the assay, which act as insulin sensitizers and are useful for treating diseases or disorders caused by insulin resistance to glucose uptake.

Some of these disorders are type 2 diabetes, obesity, glucose intolerance, dyslipidemia, hyperinsulinemia, atherosclerotic disease, polycystic ovary syndrome, coronary artery disease, hypertension, aging, non alcoholic fatty liver disease, infections, cancer and stroke.

These and other features and advantages of the present invention will be apparent to those skilled in the art from the accompanying Figures and the following description.

## BRIEF DESCRIPTION OF DRAWINGS

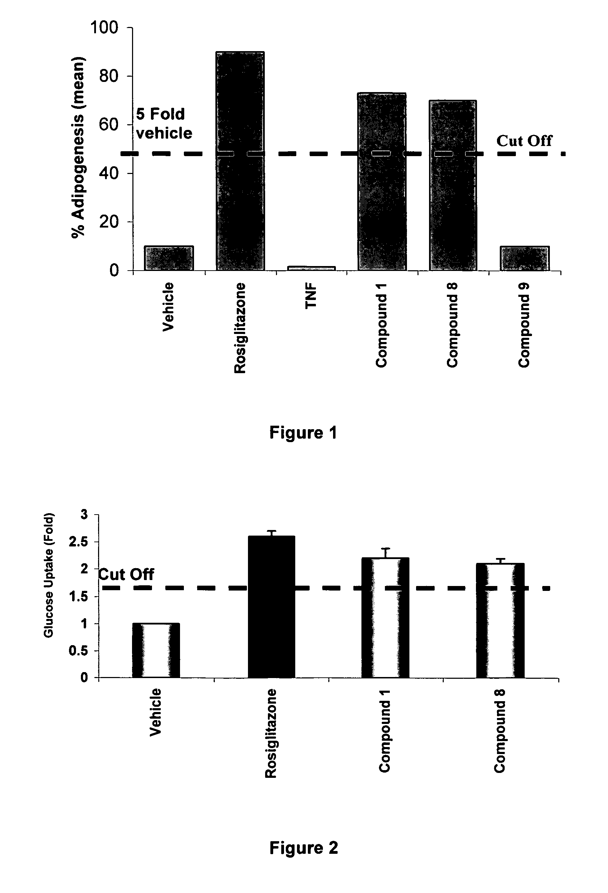

Figure 1: Effect of compounds in primary screen.

Primary screening of Compounds 1, 8 and 9: Compound 1 and Compound 8 demonstrated > 5 fold more adipogenesis than vehicle and were considered as actives. Rosiglitazone was used as a standard and TNF (Tumor Necrosis Factor) was used as negative control. Compound 9 was inactive in the adipogenesis assay.

Figure 2: Effect of adipogenesis actives in insulin resistance assay.

Compounds 1 and Compound 8, which were identified as actives in the first screen exhibited > 1.5 fold increase in glucose uptake in the second screen, the insulin resistance assay, and were considered active. Rosiglitazone was used as a standard.

Figure 3: Effect of adipogenesis inactives in insulin resistance assay.

Rosiglitazone was used as a standard. Compound 9, which was inactive in the primary screening assay, was inactive in the insulin resistance assay and did not exhibit increased glucose uptake.

Figure 4: Effect of Compound 6 on cumulative food intake of diet induced obese mice.

Diet induced obese (DIO) mice were treated with either standard (Sibutramine) or Compound 6 for 10 days. Both standard and Compound 6 significantly inhibited food intake as depicted in the graph.

Figure 5: Effect of compound of example 6 on body weight in diet induced obese mice.

Cumulative body weight gain of diet induced obese (DIO) mice treated with either standard (Sibutramine) or Compound 6 for 10 days is indicated. Both standard (Sibutramine) and Compound 6 inhibited body weight gain on all days as compared to the vehicle-treated controls.

## DETAILED DESCRIPTION OF INVENTION

Definitions:

The following is a list of definitions for terms used herein. These definitions apply to the terms as they are used throughout the specification unless otherwise limited in specific instances.

The term "insulin resistance" refers to a condition in which the tissues of the body become resistant to the effects of insulin, that is, the normal response to a given amount of insulin is reduced.

The term "insulin sensitizers" refers to agents that reduce insulin resistance and increase glucose uptake into peripheral tissue which results in decreased levels of circulating insulin.

The term "glucose uptake" refers to the measurement of glucose entry into cells. The term "lead compound" includes the meaning that the compound has desirable characteristics as an insulin-sensitizer.

"Test compounds" can be of any nature, including, chemical and natural compounds obtained from in-house library of compounds.

The term "sensitivity" refers to the ratio of the number of true *in vivo* active compounds to the sum of number of actives identified by insulin resistance assay (IR assay) and number of negatives identified in insulin resistance assay.

$$\text{Sensitivity} = \frac{\text{No. of true } \textit{in vivo} \text{ actives}}{\text{No. of actives in IR assay} + \text{No. of negatives in IR assay}} \times 100$$

The term "specificity" refers to ratio of the number of inactives in *in vivo* screen to the sum of number identified as inactives in insulin resistance assay and false positives in insulin resistance assay.

$$\text{Specificity} = \frac{\text{No. of inactives in } \textit{in vivo} \text{ screen}}{\text{No. of inactives in IR assay} + \text{No. of false positives in IR assay}} \times 100$$

The term "positive predictive value" refers to the ratio of the number of true *in vivo* actives to the sum of number of true actives in insulin resistance assay and number of false positives in insulin resistance assay.

$$\text{Positive Predictive value} = \frac{\text{No. of true } \textit{in vivo} \text{ actives}}{\text{No. of true actives in IR assay} + \text{No. of false positives in IR assay}} \times 100$$

The term "therapeutically effective amount" as used herein is meant to describe an amount of a compound identified according to the present invention effective in producing the desired therapeutic response in a particular patient suffering from a disease or disorder caused by insulin resistance to glucose uptake. Some of these disorders are type 2 diabetes, obesity, glucose intolerance, dyslipidemia, hyperinsulinemia, atherosclerotic disease, polycystic ovary syndrome, coronary artery disease, hypertension, aging, non alcoholic fatty liver disease, infections, cancer and stroke.

**The Present Methods:**

An embodiment of the present invention provides a method of identifying compounds which act as insulin-sensitizers, from among a plurality of test compounds. This method can include screening test compounds sequentially in two assays of insulin sensitivity.

An embodiment of the method of the present invention is schematically depicted in the scheme herein below. In the embodiment illustrated in the scheme, test compounds are first subjected to screening using a phenotype-based assay. The actives identified in the phenotype-based assay are further tested for insulin sensitization in a second assay using the functional endpoint of glucose entry into insulin resistant cells.

The method can include a first and a second screening step. The first screen can include screening test compounds in a phenotype-based assay and the second screen can include screening the compounds identified as active in the phenotype-based assay in an insulin resistance (IR) model.

## Scheme

3T3-L1 Fibroblast

test compounds ← Primary screening(Phenotype-based screen)

Actives           Inactives

IR-Model (IR Adipocytes)

← Secondary screening (Functional screen)

Active     Inactive

(glucose uptake)

The phenotype-based assay used for screening the test compounds in the first screening step is an adipogenesis assay. Any of a variety of adipogenesis assays can be employed. Suitable adipogenesis assays can employ fibroblasts 3T3-L1, which can be derived from mouse and insulin, for example, of either human, porcine or bovine origin. The test compounds can be dissolved at a suitable concentration (e.g., 20 $\mu$g/ml) in a suitable solvent such as DMSO. Fibroblasts can be grown in 96-well plates containing sufficient cells, for example $5 \times 10^4$ cells/well, that are cultured in nutrient medium along with 5 $\mu$g/ml of insulin. After 3 days of incubation in $CO_2$ incubators at 37 °C, the nutrient medium with compounds is removed and fresh medium is exposed to the cells. After 8 days, the ability of compounds to potentiate insulin effect on transformation into fat filled cells, adipocytes, was assessed by observing under an inverted microscope. The lipid droplets are stained with Oil red O and percentage of stained cells is determined (e.g., estimated).

Compounds that caused the formation of adipocytes by more than 5 times the vehicle are termed as actives. Compounds that did not cause any change in cell morphology are termed as inactives in this assay.

According to the method of the present invention, other phenotype-based assays such as zebrafish, Drosophila or target specific HTS screens can also be used for screening the test compounds in the primary screening step.

The zebra fish assay involves exposing the zebrafish embryos to test compounds and monitor the development without any abnormality (Current opinion in Biotechnology, 15:564-571, 2004).

In the Drosophila assay test compounds are exposed to Drosophila eggs and compounds that enhance the growth into normal flies are termed as active. (Mutation Research, 586, 115-123, 2005).

High-throughput screening, often abbreviated as HTS, is typically used to screen huge libraries of compounds for biological activity. The actives from phenotype-based assays can be tested in an IR assay.

The test compounds which are identified as actives in the phenotype-based assay (e.g., the adipogenesis assay), are then subjected to the second screen, an insulin resistance assay. The insulin resistance assay used according to the method of the present invention can be an *in vitro* model - tissue exhibiting insulin resistance. Any of a variety of insulin resistance assays can be employed. The ability of compounds to improve insulin action on glucose entry into the insulin resistant cell can be determined in comparison to the cells which have only insulin.

A suitable insulin resistance assay includes screening of compounds which act as insulin-sensitizers using chronically dexamethasone treated 3T3-L1 adipocytes as an *in vitro* model. This is a known model for studying insulin signaling and insulin resistance. Chronically dexamethasone treated 3T3-L1 adipocytes serve as a valid *in vitro* model of insulin resistance resembling diabetes patients.

In an embodiment, the method according to the present invention includes screening compounds identified as active in the phenotype-based assay against dexamethasone treated insulin resistant adipocytes. For example, the method can include applying actives from the insulin sensitivity assay at a concentration of 1-10 μM to the insulin resistant adipocytes for 1-4 days. The adipocytes were previously prepared for the assay by long term exposure to dexamethasone, for example, at 100 nM for about 24-48 hours.

The method can also include determining whether the compounds enhance glucose uptake in the insulin resistant adipocytes by more than 50% above that enhanced by insulin. The method can then include selecting compounds as active, if they caused 1.5 fold or more increase in glucose uptake compared to insulin treated adipocytes. Glucose uptake can be measured by any of a variety of known methods, such as measuring uptake of $^{14}$C-labeled 2-deoxyglucose, a non metabolisable analogue of D-Glucose.

In an embodiment of the present invention, the method can include selecting those compounds determined to be actives in the two assays as lead compounds for treating diseases caused by insulin resistance to glucose uptake and related disorders. Some of these disorders are type 2 diabetes, obesity, glucose intolerance, dyslipidemia, hyperinsulinemia, atherosclerotic disease, polycystic ovary syndrome, coronary artery disease, hypertension, aging, non alcoholic fatty liver disease, infections, cancer, and stroke.

The present method can also include screening the leads for activity in an animal model of insulin resistance, such as in leptin resistant genetically diabetic *db/db* mice. These mice develop obesity and hyperinsulinemia and hyperglycemia from seven weeks of age and maintain diabetic phenotype up to twelve weeks of age.

In an embodiment, the method includes selecting 6-8 weeks old male *db/db* mice based on blood glucose levels determined after withholding feed for four hours. For example, the method can employ animals with plasma glucose levels in a suitable range, such as 300-500 mg/dl. The selected mice can be orally dosed with test (lead) compound, for example, in 0.5% carboxy methyl cellulose (CMC) vehicle at a suitable frequency and for a suitable time, such as twice daily for 10 consecutive days. This embodiment of the method can include obtaining blood on, for example, day 5 and day 10, after withholding feed for four hours, and determining plasma glucose levels. These levels can be determined using enzymatic methods (e.g., Diasys kit, Germany) in an autoanalyser. Lead compounds are considered to be *in vivo* actives if they produce statistically significant fall in plasma glucose on day 10 compared to mice treated with 0.5% CMC vehicle. Rosiglitazone can be used as a standard at 5 mg per kg (mpk) bid for comparison with test compounds.

The present invention also relates to a method for treating insulin resistant Type 2 diabetes mellitus. The method of treating can include providing an *in vivo* active compound from the present screening methods. The method of treating also includes administering the *in vivo* active to a patient suffering from a disease caused by insulin resistance to glucose uptake. The method of treating can be employed on a subject even prior to the onset of elevated blood glucose levels.

The present method can also include screening the leads for activity in an animal model such as in a chronic study using diet induced obese (DIO) mice.

In an embodiment, the method includes selecting 17-18 week old male diet induced obese (DIO) mice, which were maintained on a high fat diet (D12451, Research Diets Inc, New Brunswick, NJ 08901, USA, 45% kcal from fat), based on body weight. The selected mice can be dosed intraperitoneally with test (lead) compound, for example, in 0.5% CMC vehicle at a suitable frequency and for a suitable time, such as once daily for 10 consecutive days. This embodiment of the method can include recording body weight and food weight daily. Lead compounds are considered to be *in vivo* active if they produce statistically significant decrease in cumulative food intake for up to 1 day and a statistically significant decrease in cumulative body weight gain on day 10 as compared to mice treated with the 0.5% CMC vehicle. Sibutramine can be used as a standard at 3 mpk for comparison.

The present invention also relates to a method for treating obesity. The method of treating can include providing an *in vivo* active compound from the present screening methods. The method of treating also includes administering the *in vivo* active to a patient suffering from obesity, a disease caused by insulin resistance.

The present method can also include screening the leads for activity in an animal model using *db/db* mice for evaluation of lipid levels.

Groups of male *db/db* mice were orally dosed twice a day (bid) for a period of fifteen days, with either the vehicle or test compound (5 mpk, 25 mpk, 50 mpk, 100 mpk and 200 mpk) or with the standard drug, Rosiglitazone (5 mpk). Body weight was measured daily. On day 15, the animals were deprived of food for 4 hours after the last dose administration. Blood was collected at the end of the 4-hour period using heparinised capillaries by a retro-orbital puncture. Plasma samples were analyzed for, triglyceride and cholesterol, using the autoanalyser.

The present invention also relates to a method for treating dyslipidemia. The method of treating can include providing an *in vivo* active compound from the present screening methods. The method of treating also includes administering the *in vivo* active to a patient suffering from elevated levels of cholesterol and triglycerides.

Suitable compounds identified according to the method of the present invention (i.e., *in vivo* actives) include the compounds listed below, or pharmaceutically acceptable salts or solvates or crystalline forms thereof, selected from but not limited to:

2-{4-[2-(tert-Butoxycarbonyl-methyl-amino)-ethoxy]-benzyl}-2-methoxy-malonic acid dimethyl ester;
3-{4-[2-(tert-Butoxycarbonyl-methyl-amino)-ethoxy]-phenyl}-2-methoxy-propionic acid;
3-{5-[2-(Benzooxazol-2-yl-methyl-amino)-ethylamino]-1-oxo-1,3-dihydro-isoindol-2-yl}-propionic acid ethyl ester;
2,4-Dichloro-N-[5-chloro-6-(isoquinolin-3-yloxy)pyridin-3-yl]-benzenesulfonamide; N-{6-[4-(4-Acetyl-piperazin-1-yl)-phenoxy]-5-chloro-pyridin-3-yl}-2,4-dichlorobenzene sulfonamide; and
N-[5-Chloro-6-(quinolin-3-yloxy)-pyridine-3-yl]-4-methoxy-benzenesulfonamide.

The present invention also relates to *in vivo* actives identified by the screening method of the present invention. The present compounds include those listed above. The examples as described below are given by way of illustration only and are not to be construed as limiting the invention in any way in as much as many variations of the invention are possible within the meaning of the invention.

## EXAMPLES

**List of abbreviations:**

DMSO : Dimethyl sulfoxide
CPM : Counts per minute
mpk : mg per Kg.
od : Once a day
bid : Twice a day
HEPES : N-(2-hydroxyethyl)-piperazine-N'-2-ethanesulfonic acid
CMC : carboxy methyl cellulose

**Example 1**

**2-{4-[2-(tert-Butoxycarbonyl-methyl-amino)-ethoxy]-benzyl}-2-methoxy-malonic acid dimethyl ether (Compound 1)**

The title compound was prepared in four steps starting from 4-hydroxy-benzaldehyde as follows.

Step 1

Preparation of 4-hydroxymethyl-phenol

4-Hydroxy-benzaldehyde (20 g, 0.16 mol) was dissolved in methanol (150 mL) and to it sodium borohydride (7.4 g, 0.19 mol) was added at 0 °C in portions (approximately 500 mg each time) over a period of 30 minutes with stirring. Stirring was continued for another 30 minutes. Solvent was evaporated and water was added to it. The reaction mixture was acidified using 10 % acetic acid in water and was extracted using ethyl acetate. Ethyl acetate layer was washed with water and brine and was dried over sodium sulfate. Solvent was removed and crude product was crystallized using pet ether and ethyl acetate to obtain the title compound.
Yield: 18 g (88.5 %).

Step 2

Preparation of acetic acid 4-hydroxy-benzyl ester

The compound of step 1 (12.5 g) was acetylated using acetic anhydride (25 mL) and boron trifluoride diethyl etherate (4.5 mL) in tetrahydrofuran with stirring at 0 °C for 2 hours. The reaction mixture was neutralized using sodium bicarbonate and was extracted using ethyl acetate. Ethyl acetate layer was washed with water and brine and was dried over sodium sulfate. Solvent was removed and crude product was purified by column chromatography (silica gel - -200 mesh, 10 % ethyl acetate in pet ether) to obtain the title compound.
Yield: 9.0 g (53.8 %); $^1$H NMR (CDCl$_3$, 300 MHz): δ 2.0 (s, 3H), 5.0 (s, 2H), 6.8 (d, 2H), 7.25 (d, 2H).

Step 3

Preparation of 2-(4-hydroxy-benzyl)-2-methoxy-malonic acid dimethyl ester

Compound of step 2 (9.0 g, 0.054 mol) and methoxy dimethyl malonate (8.8 g, 0.054 mol) were dissolved in dimethylformamide (125 mL) and stirred. To this reaction mixture, cesium carbonate (17.65 g, 0.054 mol) was added and mixture was stirred at 60 °C for 2 hours. Reaction mixture was diluted with water and was extracted with ethyl acetate. Ethyl acetate layer was washed with water and brine and was dried over sodium sulfate. Solvent was removed and crude product was purified by column chromatography (silica gel - ~200 mesh, 10-20 % ethyl acetate in pet ether) to obtain the title compound.

Yield: 11.8 g (81.3 %); $^1$H NMR (CDCl$_3$, 300 MHz): δ 3.2 (s, 2H), 3.4 (s, 3H), 3.7 (s, 6H), 6.7 (d, 2H), 7.0 (d, 2H); MS (ES$^-$): (m/z) 267 (M$^+$ -1).

Step 4

Preparation of 2-{4-[2-(tert-butoxycarbonyl-methyl-amino)-ethoxy]-benzyl}-2-methoxy -malonic acid dimethyl ester

Compound of step 3 (11 g, 0.0374 mol) and (2-hydroxy-ethyl)-methyl carbamic acid tert-butyl ester (9.82 g, 0.0561) were dissolved in dry tetrahydrofuran (115 mL) and triphenylphosphine (15.7 g, 0.0598 mol) was added. The reaction mixture was stirred at 0 °C for 5 minutes. A solution of DEAD (diethyl azodicarboxylate, 10.41 g, 0.0598 mol) in tetrahydrofuran was added slowly to this mixture over a period of 30 minutes. Stirring was continued for another 20 hours at 25 °C. Solvent was removed, residue was dissolved in diethyl ether and to it hexane was added. Resulting white precipitate was filtered. The crude product was purified by column chromatography (silica gel - -200 mesh, 10-20 % ethyl acetate in pet ether) to obtain the title compound.

Yield: 10.6 g (60.77 %); $^1$H NMR (CDCl$_3$, 300 MHz): δ 1.46 (s, 9H), 2.97 (s, 3H), 3.3 (s, 2H), 3.4 (s, 3H), 3.5 (t, 2H), 3.7 (s, 6H), 4.0 (t, 3H); MS (ES$^+$): m/z: 448 (M$^+$ + Na$^+$).

**Example 2**

**3-{4-[2-(tert-Butoxycarbonyl-methyl-amino)-ethoxy]-phenyl}-2-methoxypropionic acid (Compound 2)**

Compound of example 1 (4.0 g, 0.0094 mol) was dissolved in ethanol (30 mL) and to it 1 N sodium hydroxide solution (23.5 mL) was added and mixture was stirred at 25 °C for 1 hour. The reaction mixture was concentrated and was diluted with water. Reaction mixture was acidified with dilute hydrochloric acid and was extracted with ethyl acetate. Ethyl acetate layer was washed with water and brine and was dried over sodium sulfate. Solvent was removed and semi-pure compound (3.3 g) obtained was dissolved in DMSO (10 mL). Mixture was heated at 60 °C with stirring for 12 hours. The reaction mixture was diluted with water and was extracted with ethyl acetate. Ethyl acetate layer was washed with water and brine and was dried over sodium sulfate. Solvent was removed and crude product obtained was purified by column chromatography (silica gel - ~200 mesh, 2 % methanol in chloroform) to obtain the title compound.

Yield: 1.5 g (45.45 %); IR (KBr, cm$^{-1}$): 3468, 2976, 1739, 1693; $^1$H NMR (CDCl$_3$, 300 MHz): δ 1.4 (s, 9H), 3.0 (s, 3H), 3.4 (s, 3H), 3.5 (t, 2H), 3.9. (dd, 1H), 4.0 (t, 2H), 6.8 (d, 2H), 7.2 (d, 2H); MS (ES$^-$): m/z 352 (M$^+$-1).

**Example 3**

**{4-[2-(1-Oxo-5-thiocarbamoyl-1,3-dihydro-isoindol-2-yl)-acetyl]-phenoxy}-aceticacid-2-methyl-2-(pyridine-3-sulfonylamino)-propyl ester (Compound 3)**

The title compound was prepared in five steps starting from 2-amino-2-methylpropane-1-ol and pyridine-3-sulfonyl chloride as follows.

Step 1

Preparation of pyridine-3-sulfonic acid (2-hydroxy-1,1-dimethyl-ethyl)-amide

2-Amino-2-methyl-propane-1-ol (7.66 g, 80 mmol) was dissolved in dichloromethane (160 mL) and pyridine (6.44 mL, 80 mmol) at 0 °C. and to this solution, pyridine-3-sulfonyl chloride (10.66 g, 60 mmol) was added in 10 equal portions over a period of 30 minutes. The reaction mixture was stirred at 0 °C for 1 hour followed by stirring at 25 °C for 16 hours. The reaction mixture was diluted with dichloromethane (100 mL) and washed with water (3 x 20 mL) followed by brine (10 mL). Dichloromethane layer was dried over anhydrous sodium sulfate. Solvent was removed and the residue was purified by column chromatography (silica gel - ~200 mesh, 10 % acetonitrile in chloroform followed by 5 % methanol in chloroform) to obtain the title compound.

Yield: 5.94 g (30 %); IR (KBr, cm$^{-1}$): 3300 (br), 1580 (br), 1425, 1330; $^1$H NMR (CDCl$_3$, 300 MHz): δ 1.20 (s, 6H), 3.50 (s, 2H), 7.48 (m, 1H), 7.54 (m, 1H,), 8.19 (m, 1H), 8.77 (m, 1H), 9.12 (br, 1H); MS (CI): m/z 231 (M$^+$+1).

Step 2

Preparation of [4-(2-tert-butoxycarbonylamino-acetyl)-phenoxy]-acetic acid-2-methyl-2-(pyridine-3-sulfonylamino)-propyl ester

Solution of [4-(2-tert-butoxycarbonylamino-acetyl)-phenoxy]-acetic acid (5.56 g, 18 mmol) in ethyl acetate (40 mL), was chilled at 0 °C, N,N'-dicyclohexyl carbodiimide (DCC), (4.12 g, 20 mmol) in ethyl acetate (10 mL) was added with vigorous stirring. After 10 minutes, compound of step 1 (5.0 g, 20.86 mmol) dissolved in ethyl acetate (30 mL) was added followed by 4-dimethylaminopyridine (DMAP) (1.1 g, 9 mmol). The resulting reaction mixture was stirred at 0 °C for 1 hour and at 25 °C for 16 hours. The precipitated dicyclohexyl urea (DCU) was filtered off. The filtrate was washed with water (3 x 20 mL) and brine (10 mL) and was dried over sodium sulfate. Solvent was removed and the residue was purified by column chromatography (silica gel - ~ 200 mesh, 10 % acetonitrile in chloroform followed by 5 % methanol in chloroform) to obtain the title compound.

Yield: 5.72 g (61 %); IR (KBr, cm$^{-1}$): 3000, 765 (br), 1710 (br), 1680 (br), 1605, 1510; $^1$H NMR (CDCl$_3$, 300 MHz): δ1.20 (s, 6H), 1.47 [s, 9H], 4.40 (s, 2H), 4.58 (d, 2H), 4.73 (s, 2H), 5.21 (s, 1H), 5.55 (br t, 1H), 6.96 (d, 2H), 7.43 (dd, 1H), 7.93 (d, 2H), 8.16 (dd, 1H), 8.78 (dd, 1H), 9.10 (d, 1H).

Step 3

Preparation of 2-{4-[2-methyl-2-(pyridine-3-sulfonylamino)-propoxycarbonylmethoxy]-phenyl}-2-oxo-ethyl-ammonium, chloride

The compound of step 2, (9.4 g, 18.02 mmol) was dissolved in 98-100% formic acid (40 mL) and anisole (1.96 mL, 18.02 mmol) and the mixture was kept at 25 °C for 4 hours and ethereal hydrochloric acid (30 mL) was added. The ether was removed in a rotary evaporator using line vacuum followed by formic acid using high vacuum. The residue was triturated with dry ether to obtain the title compound.

Yield: 8.0 g (97%).

Step 4

Preparation of {4-[2-(5-cyano-1-oxo-1,3-dihydro-isoindol-2-yl)-acetyl]-phenoxy}-acetic acid 2-methyl-2-(pyridine-3-sulfonylamino)-propyl ester

Compound of step 3 (8 g, 17 mmol) was dissolved in dimethylformamide (100 mL) and dichloromethane (50 mL) and solution was cooled at -30 °C. To this solution sodium bicarbonate (14.21 g, 170 mmol) was added followed by drop-wise addition of 2-bromomethyl-4-cyano benzoic acid ethyl ester (4.021 g, 15.45 mmol) dissolved in dichloromethane (50 mL) over a period of 45 minutes. After the addition, stirring was continued for 16 hours at 25 °C. The reaction mixture was diluted with dichloromethane (200 mL) and water (1 L). The organic layer was separated and was washed with water (3 x 50 mL) followed by brine (20 mL) and was dried over anhydrous sodium sulfate. Solvent was removed and the residue was purified by column chromatography (silica gel - ~ 200 mesh, 10 % acetonitrile in chloroform followed by 2 % methanol in chloroform) and finally crystallized from hot ethyl acetate-petroleum ether to obtain the title compound.

Yield: 2.91 g (30 %); mp:190-92 °C; $^1$H NMR (DMSO-d$_6$, 300 MHz): δ 1.08 (s, 6H), 4.00 (s, 2H), 4.57 (s, 2H), 4.88 (s, 2H), 5.13 (s, 2H), 7.07 (d, 2H), 7.62 (m, 1H), 7.88 (d, 1H), 8.03 (m, 4H), 8.77 (m, 1H), 8.98 (d, 1H); MS (ES$^+$): m/z 563 (M$^+$+1).

Step 5

Preparation of {4-[2-(1-oxo-5-thiocarbamoyl-1,3-dihydro-isoindol-2-yl)-acetyl]-phenoxy}-acetic acid 2-methyl-2-(pyridine-3-sulfonylamino)-propyl ester

Compound of step 4, (2.6 g, 4.62 mmol), was dissolved in pyridine (100 mL) and triethyl amine (20 mL). Dry hydrogen sulphide gas was bubbled for 1 hour at 25 °C. The resulting deep brown solution was stirred at 25 °C for additional 1 hour. The solvent was evaporated in a rotary evaporator under vacuum. The crude product was purified by column chromatography (silica gel - ~ 200 mesh, 10 % acetonitrile in chloroform followed by 3 % methanol in chloroform). The pure fraction obtained was triturated with chloroform (5 mL), diethyl ether (20 mL) and methanol (5 mL). The pale yellow solid was filtered and washed with chloroform-ether to obtain the title compound.

Yield: 1.8 g (65 %); mp: 197-98 °C; IR (KBr), cm$^{-1}$: 3383, 3300, 3224, 2920, 1755, 1677, 1633, 1600, 1572; $^1$H NMR (DMSO-d$_6$, 300 MHz): δ 1.08 (s, 6H), 3.99 (s, 2H), 4.53 (s, 2H), 4.87 (s, 2H), 5.10 (s, 2H), 7.06 (d, 2H), 7.62 (m, 1H), 7.72 (d, 1H), 7.92 (d, 1H), 8.03 (m, 3H), 8.07 (s, 1H), 8.20 (br 1H), 8.78 (m, 1H), 8.98 (d, 1H), 9.68 and 10.06 (2 x s, 2H); MS (ES$^+$:): m/z 597 (M$^+$+ 1); Analysis calculated for $C_{28}H_{28}N_4O_7S_2$: C, 56.36; H, 4.73; N, 9.39; S, 10.75 %; found, C, 56.05; H, 4.64; N, 8.72; S, 10.46 %.

**Example 4**

**3-{5-[2-(Benzooxazol-2-yl-methyl-amino)-ethylamino]-1-oxo-1,3-dihydro-isoindol-2-yl}propionic acid ethyl ester (Compound 4)**

The title compound was prepared in three steps starting from 2-bromomethyl-4-nitrobenzoic acid ethyl ester as follows.

Step 1

Preparation of 3-(5-nitro-1-oxo-1,3-dihydro-isoindole-2-yl)-propionic acid ethyl ester

A solution of 2-bromomethyl-4-nitro-benzoic acid ethyl ester (5 g, 17.36 mmol) in dichloromethane (100 mL) was added dropwise to a suspension of β-alanine ethyl ester hydrochloride (βAla-OEt.HCl) (4 g, 26.05 mmol) and sodium bicarbonate (10.94 g, 130 mmol) in dimethylformamide (100 mL) at -20 °C for 30 min. with vigorous stirring. After the addition was over, the reaction mixture was brought to 25 °C and the stirring was continued for 16 hours. The solid was filtered. The filtrate was diluted with additional dichloromethane (100 mL) and diluted with water (500 mL). The organic layer was separated and washed with water (3x 50 mL) and was dried over anhydrous sodium sulfate. The solvent was removed and the residue was purified by column chromatography (silica gel, 1-10 % acetonitrile in chloroform). The semi-pure compound was crystallized using chloroform and petroleum ether to obtain the title compound.

Yield: 3.0 g; $^1$H NMR (CDCl$_3$, 300 MHz); δ 1.24 (t, 3H), 2.76 (t, 2H), 3.93 (t, 2H), 4.18 (q, 2H), 4.61 (s, 2H), 7.97-8.32 (br, 3H); MS (EI): m/z 278 (M$^+$).

Step 2

Preparation of 3-(5-amino-1-oxo-1,3-dihydro-isoindole-2-yl)-propionic acid ethyl ester Compound of step 1, (2.9 g, 10.4 mmol) was dissolved in ethanol (50 mL) and dimethylformamide (40 mL). To this solution, ammonium chloride solution (8.92 g, 166.8 mmol) and Fe (5.84 g) were added and the reaction mixture was heated at 75 °C for 3 hours. The solution was cooled to 25 °C and was filtered. The filtrate was evaporated in a rotary evaporator using vacuum. The residue was dissolved in ethyl acetate (50 mL) and 10 % sodium bicarbonate (30 mL) and the mixture was vigorously stirred for 30 minutes. The ethyl acetate layer was separated and washed with water (3 x 10 mL) and was dried over anhydrous sodium sulfate. The solvent was removed. The residue was purified by column chromatography (silica gel ~ 200 mesh, 10 % acetonitrile in chloroform followed by 2 % methanol in chloroform) to obtain the title compound.

Yield: 1.6 g; $^1$H NMR (CDCl$_3$, 300 MHz): δ 1.23 (t, 3H), 2.68 (t, 2H), 3.84 (t, 2H), 4.13 (q, 2H), 4.32 (s, 2H), 6.63-6.69 (br, 3H); MS (EI): m/z 248 (M$^+$).

Step 3

Preparation of 3-{5-[2-(benzooxazol-2-ylmethyl-amino)-ethylamino]-1-oxo-1,3-dihydro-isoindole-2-yl}-propionic acid ethyl ester

A solution of compound obtained in step 2 (0.5 g, 2.03 mmol), methanesulfonic acid 2-(benzooxazol-2-ylmethyl-amino)-ethyl ester (0.82 g, 3 mmol) and triethyl amine (0.44 mL, 3 mmol) in dimethylformamide (2 mL) was heated at 80 °C for 40 hours. The solvent was removed and the residue was dissolved in ethyl acetate (20 mL). the ethyl acetate layer was washed with water (3 x 5 mL) and dried over anhydrous sodium sulfate. Solvent was evaporated and the residue was purified by column chromatography (silica gel - ~ 200 mesh, 10 % acetonitrile in chloroform followed by 2 % isopropanol in chloroform) to obtain the title compound.

Yield: 0.42 g; $^1$H NMR (CDCl$_3$, 300 MHz): δ 1.19 (t, 3H), 2.66 (t, 2H), 3.32 (s, 2H), 3.63 (t, 2H), 3.77 (m, 4H), 4.12 (q, 2H), 4.25 (s, 2H), 6.49 (t, 1H), 6.58 (t, 1H), 6.77-6.86 (br, 4H), 7.29 (d, 1H), 7.90 (s, 1H).

**Example 5**

**2,4-Dichloro-N-[5-chloro-6-(isoquinolin-3-yloxy)pyridin-3-yl]-benzene sulfonamide (Compound 5)**

The title compound is prepared in five steps from 2-hydroxy-5-nitro pyridine as described herein.

Step 1

Preparation of 2-hydroxy-3-chloro-5-nitro pyridine

2-Hydroxy-5-nitro-pyridine (1 g, 7.14 mmol) was added in portions to concentrated hydrochloric acid (4.5 mL) with constant stirring and was heated to 50 °C. A solution of sodium chlorate (266 mg, 2.5 mmol) in water (4 mL) was added slowly to this mixture. The reaction was maintained at the same temperature for 1 hour, and was cooled to 0 °C. The precipitate obtained was filtered, washed with water and dried to obtain the title compound.
Yield: 0.850 g (68.2%); $^1$H NMR (DMSO-d$_6$, 300 MHz): δ 8.36 (d, 1H), 8.65 (d, 1H).

Step 2

Preparation of 2,3-dichloro-5-nitro pyridine

Quinoline (0.3 mL, 2.34 mmol) was added to phosphorusoxychloride (POCl$_3$) (0.5 mL 4.68 mmol) at 0 °C under nitrogen atmosphere. To this stirred mixture, 2-hydroxy-3-chloro-5-nitro pyridine (816 mg, 4.68 mmol) (as obtained in step 1) was added. The reaction mixture was heated at 120 °C for 2 hours, cooled to 0 °C followed by addition of ice-cold water. The precipitate obtained was filtered, washed with water and dried to obtain the title compound.
Yield: 0.630 g (70.3 %); $^1$H NMR (DMSO-d$_6$, 300 MHz): δ 8.94 (d, 1H), 9.16 (d, 1H).

Step 3

Preparation of 3-(3-chloro-5-nitro-pyridin-2-yloxy)-isoquinoline

Dry dimethylformamide (10 mL) was added to 3-hydroxy-isoquinoline (459 mg, 3.16 mmol) under stirring and cesium carbonate was added (1.03 g, 3.16 mmol) at room temperature (25 °C). After 30 minutes 2,3-dichloro-5-nitro pyridine (610 mg, 3.16 mmol) (as obtained in step 2), was added and the stirring was continued further for 18 hours. The solvent was removed under vacuum and to the resulting mass was added water (20 mL), extracted with ethyl acetate, dried over sodium sulfate and concentrated under vacuum to obtain crude 3-(3-chloro-5-nitro-pyridin-2-yloxy)-isoquinoline that was further purified by column chromatography (silica gel- ~ 200 mesh, gradient 5-30 % ethyl acetate in petroleum ether) to obtain the title compound. Yield: 911 mg (96%)
$^1$H NMR (DMSO-d$_6$, 300 MHz): δ 7.68 (t, 1H), 7.82 (m, 2H), 8.01 (d, 1H), 8.21 (d, 1H), 8.94 (s, 2H), 9.20 (s, 1H).

Step 4

Preparation of 5-chloro-6-(isoquinolin-3yloxy)-pyridin-3-yl amine

Compound of step 3 (2.51 g, 8.34 mmol) was dissolved in ethyl acetate (50 mL). Stannous chloride dihydrate (7.52 g, 33.36 mmol) was added at room temperature and stirring was continued further for 18 hours. Solvent was removed under vacuum and chloroform (50 mL) was added. 1 N sodium hydroxide solution was added until a clear solution was obtained. Organic layer was separated and extracted with chloroform. The chloroform layer was washed with water & brine, dried over sodium sulfate and concentrated under vacuum to obtain crude product was further purified by column chromatography (silica gel -120 mesh, gradient 20-40 % ethyl acetate in petroleum ether) to obtain the title compound.
Yield:1.85 g (81.5 %); $^1$H NMR (DMSO-d$_6$, 300 MHz): δ 5.49 (s, 2H), 7.21 (d, 1H), 7.32 (s, 1H), 7.54 (m, 2H), 7.70 (t, 1H), 7.90 (d, 1H), 8.07 (d, 1H), 9.00 (s, 1H),

Step 5

Preparation of 2,4-Dichloro-N-[5-chloro-6-(isoquinolin-3-yloxy)-pyridin-3-yl]-benzene sulfonamide

5-chloro-6-(isoquinolin-3yloxy)-pyridin-3-yl amine (272 mg, 1 mmol) (compound of step 4) was dissolved in dry dichloromethane (15 mL) and 2,4-dichloro sulfonyl chloride (246 mg, 1 mmol) was added, followed by the addition of pyridine (1 mmol). The reaction mixture was heated at 45 °C for 15 hours, cooled to room temperature and was diluted with dichloromethane (25 mL), washed with water and dried over sodium sulfate. Solvent was removed and crude product, which purified by column chromatography (silica gel - ~200 mesh, gradient 10-25 % ethyl acetate and petroleum ether) to obtain the title compound.

Yield: 337 mg (70%); $^1$H NMR (DMSO-$d_6$, 300 MHz); δ 7.57 (s, 1H), 7.61 (m, 2H), 7.74 (m, 2H), 7.84 (d, 1H), 7.91 (d, 2H), 8.02 (d, 1H), 8.09 (d, 1H), 9.05 (s, 1H), 11.12 (s, 1H); MS (ES[+]): m/z 481.94 (M[+] +1).

**Example 6**

**N-{6-[4-(4-Acetyl-piperazin-1-yl)-phenoxy]-5-chloro-pyridin-3-yl}-2,4-dichloro benzenesulfonamide (Compound 6)**

The title compound was prepared in 3 steps starting from 1-{4-(-hydroxy-phenyl) piperazine-1-yl}-ethanone

Step 1

Preparation of 1-{4-[4-((3-chloro-5-nitro-pyridin-2yloxy)-phenyl]-piperazin-1-yl-ethanone

Dry dimethylformamide (10 mL) was added to 1-[4-(-hydroxy-phenyl--piperazine-1-yl]-ethanone (696 mg, 3.16 mmol) with stirring and cesium carbonate was added (1.03 g, 3.16 mmol) at room temperature (25 °C). After 30 minutes 2,3-dichloro-5-nitro-pyridine (610 mg, 3.16 mmol) (as obtained in step 2, example 5), was added and the stirring was continued further for 18 hours. The solvent was removed under vacuum and to the resulting mass was added water (20 mL), extracted with ethyl acetate, dried over sodium sulfate and concentrated under vacuum to obtain crude product that was further purified by column chromatography (silica gel - ~ 200 mesh, 30% ethyl acetate in pet ether) to obtain the title compound.

Yield: 1.09 g (92.9 %); $^1$H NMR (CDCl$_3$) δ: 2.01 (s, 3H), 2.99 (t, 2H), 3.05 (t, 2H), 3.54 (s, 4H), 5.30 (s, 2H), 6.85 (d, 1H), 6.93 (d, 1H), 7.17 (s, 1H), 7.43 (s, 1H).

Step 2

Preparation of 1-{4-[4-((5-amino-3-cholro-pyridin-2yloxy)-phenyl]-piperazin-1-yl-ethanone

Compound of step 1 (3.15 g, 8.34 mmol) was dissolved in ethyl acetate (50 mL). Stannous chloride dihydrate (7.52 g, 33.36 mmol) was added at room temperature (25 °C) and stirring was continued for 18 hours. Solvent was removed under vacuum and chloroform (50 mL) was added. 1 N sodium hydroxide solution was added until a clear solution was obtained. The organic layer was separated and extracted with chloroform. The chloroform layer was washed with brine and water successively, dried over sodium sulfate and concentrated under vacuum to obtain crude product that was further purified by column chromatography (silica gel - ~200 mesh, 1 % methanol in chloroform) to obtain the title compound.

Yield: 1.77 g (61.52 %); $^1$H NMR (CDCl$_3$) δ: 2.04 (s, 3H), 3.10 (m, 2H), 3.18 (m, 2H), 3.59 (brs, 4H), 5.32 (s, 2H), 7.05 (d, J=9Hz, 2H), 7.14 (d, J=9Hz, 2H), 8.86 (d, J=2.4 Hz, 1H), 8.95 (d, J=2.4 Hz, 1H); MS: 347 (M+1).

Step 3

Preparation of N-{6-[4-(4-acetyl-piperazin-1-yl)-phenoxy]-5-chloro-pyridin-3- yl}-2,4-dichloro-benzenesulfonamide

Compound of step 2, (346 mg, 1 mmol) was dissolved in dry dichloromethane (15 mL) and 2,4-dichloro-sulfonyl chloride (246 mg, 1 mmol) was added, followed by the addition of pyridine (1 mmol). The reaction mixture was heated at 45 °C for 15 hours, cooled to room temperature and was diluted with dichloromethane (25 mL), washed with water and dried over sodium sulfate. Solvent was removed and crude product was purified by column chromatography (silica gel - ~ 120 mesh, 40 % ethyl acetate in petroleum ether) to obtain the title compound.

$^1$H NMR (DMSO-$d_6$, 300 MHz): δ 2.02 (s, 3H), 2.50 (t, 4H), 3.05 (d, 4H), 6.94 (s, 4H), 7.57 (dd, 1H), 7.70 (d, 1H), 7.76 (d, 1H), 7.87 (d, 1H), 7.96 (d, 1H); MS (ES⁻): m/z 553 (M ⁺-1).

**Example 7**

**N-{6-[4-(4-Acetyl-piperazin-1-yl)-phenoxy]-5-chloro-pyridin-3-yl succinamic acid (Compound 7)**

1-{4-[4-(5-Amino-3-cholro-pyridin-2yloxy)-phenyl]-piperazin-1-yl-ethanone (as in step 2, example 6) (346 mg, 1.0 mmol) was dissolved in dry benzene (15 mL) and succinic anhydride (100 mg, 1.0 mmol) was added, The reaction mixture was refluxed for 10-12 hours, cooled to room temperature. Solvent was removed using vacuum and crude product was purified by column chromatography. (silica gel - ~200 mesh, gradient 20-40 % ethyl acetate in petroleum ether) to obtain the title compound.
Yield: 320 mg (75 %); ¹H NMR (DMSO-d₆, 300 MHz): δ 2.02 (s, 3H), 2.48 (t, 4H), 3.04 (d, 4H), 3.56 (s, 4H), 6.98 (s, 4H), 8.10 (s, 1H), 8.28 (s, 1H), 10.26 (s, 1H), 12.17 (s, 1H); MS (ES⁻): (m/z) 445 (M⁺-1).

**Example 8**

**N-[5-Chloro-6-(quinolin-3-yloxy)-pyridine-3-yl]-4-methoxy-benzene sulfonamide (Compound 8)**

The title compound was prepared in three steps starting from 2,3-dichloro-5-nitro pyridine and 3-hydroxy-quinoline

Step 1

Preparation of 3-(3-chloro-5-nitro-pyridin-2-yloxy)-quinoline

Dry dimethylformamide (10 mL) was added to 3-hydroxy-quinoline (459 mg, 3.16 mmol) under stirring and cesium carbonate was added (1.03 g, 3.16 mmol) at room temperature (25 °C). After 30 minutes 2,3-dichloro-5-nitro pyridine (610 mg, 3.16 mmol) (as obtained in step 2, example 5), was added and the stirring was continued further for 18 hours. The solvent was removed under vacuum and to the resulting mass was added water (20 mL), extracted with ethyl acetate, dried over sodium sulfate and concentrated under vacuum to obtain crude product that was further purified by column chromatography (silica gel - ~ 200 mesh, 1 % methanol in chloroform) to obtain the title compound.
Yield: 911 mg (96 %); ¹H NMR (DMSO-d₆, 300 MHz): δ 7.69 (t, 1H), 7.82 (t, 1H), 7.98 (d, 1H), 8.09 (d, 1H), 8.35 (d, 1H), 8.95 (d, 1H), 9.03 (d, 2H).

Step 2

Preparation of 5-chloro-6-(quinolin-3yloxy)-pyridin-3-yl amine

Compound of step 1 (2.51 g, 8.34 mmol) was dissolved in ethyl acetate (50 mL). Stannous chloride dihydrate (7.52 g, 33.36 mmol) was added at room temperature (25 °C) and stirring was continued further for 18 hours. Solvent was removed under vacuum and chloroform (50 mL) was added. 1 N sodium hydroxide solution was added until a clear solution was obtained. The organic layer was separated and extracted again with chloroform. The chloroform layer was washed with brine and water successively, dried over sodium sulfate and concentrated under vacuum to obtain crude product that was further purified by column chromatography (silica gel-- 200 mesh, 1 % methanol in chloroform) to obtain the title compound.
Yield: 1.85 g (81.5%); ¹H NMR (DMSO-d₆, 300 MHz): δ 5.49 (s, 2H), 7.26 (d, 1H), 7.49 (d, 1H), 7.60 (td, 1H), 7.70 (td, 1H), 7.85 (d, 1H), 7.92 (dd, 1H), 8.01 (d, 1H), 8.75 (d, 1H).

Step 3

Preparation of N-[5-chloro-6-(quinolin-3-yloxy)-pyridine-3-yl]-4-methoxy-benzene sulfonamide

5-Chloro-6-quinoloxy-3-azinamine (200 mg, 0.738 mmol) was dissolved in dry dichloromethane (15 mL) and 4-methoxy-sulfonyl chloride (206 mg, 0.738 mmol) was added, followed by the addition of pyridine (1 mmol). The reaction mixture was heated at 45 °C for 15 hours, cooled to room temperature (25 °C) and was diluted with dichloromethane (25 mL), washed with water and dried over sodium sulfate. Solvent was removed and crude product was purified by column chromatography (silica gel - ~200 mesh, 1 % methanol in chloroform silica gel) to obtain the title compound.
Yield: 220 mg (67.57%); ¹H NMR (DMSO-d₆, 300 MHz): δ 3.80 (s, 3H), 7.10 (dd, 2H), 7.60 (t, 1H), 7.64 (t, 1H), 7.69 (d, 2H), 7.70 (d, 1H), 7.75-7.72 (m, 2H), 7.95 (d, 1H), 8.04 (d, 1H), 8.15 (d, 1H), 10.44 (s, 1H); MS (ES$^+$): (m/z) 441 (M$^+$ + 1).

**Example 9**

**4-(Imino-methoxycarbonylaminomethyl)-2-methyl-benzoic acid ethyl ester (Compound 9)**

The title compound was prepared in three steps starting from 4-cyano-2-methylbenzoic acid ethyl ester as follows.

Step1

4-(N-Hydroxycarbamimidoyl)-2-methyl-benzoic acid ethyl ester

A mixture of 4-cyano-2-methyl-benzoic acid ethyl ester (5 g, 26.5 mmol), hydroxylamine hydrochloride (96.82 g, 98.05 mmol) and sodium carbonate (4.78 g, 45.05 mmol) in ethanol (25 mL) and water (75 mL) was refluxed for 3 hours. The solvent was evaporated. The residue was extracted with ethyl acetate (60 mL). The ethyl acetate layer was washed with water (3 x 10 mL). It was dried over anhydrous sodium sulfate. The solvent was removed. The crude product was purified by column chromatography (silica gel, 5-10 % acetonitrile in chloroform). The semi-pure compound was crystallized using ethyl acetate and petroleum ether to obtain the title compound.
Yield: 5.2 g; $^1$H NMR (CDCl$_3$, 300 MHz): δ 1.4 (t, 3H), 2.63 (s, 3H), 4.38 (q, 2H), 4.92 (s, 2H), 7.5 (d, 1H), 7.54 (s, 1H), 7.95 (d, 1H).

Step 2

Preparation of 4-carbamimidoyl-2-methyl-benzoic acid ethyl ester

The compound obtained in step 1 (5 g, 22.5 mmol) was dissolved in acetic acid (33 mL) in a hydrogenation bottle. Acetic anhydride (3.2 mL, 33.75 mmol) was added and stirred for 5 minutes. The mixture was then hydrogenated over 10 % Pd-C (0.8 g) at 10 psi for 30 minutes. The catalyst was filtered off and the filtrate was concentrated. The white solid thus obtained was triturated with dry ether to obtain the title compound.
Yield: 4.8 g; $^1$H NMR (CD$_3$OD, 300 MHz): δ 1.45 (t, 3H), 1.96 (s, 3H), 2.68 (s, 3H), 4.45 (q, 2H), 7.72 (d, 1H), 7.77 (s, 1H), 8.08 (d, 1H).

Step 3

Preparation of 4-(imino-methoxycarbonylaminomethyl)-2-methyl-benzoic acid ethyl ester

Compound of step 2, (3 g, 11.3 mmol) was dissolved in dioxane (65 mL) and water 32 mL). To this solution 1 N sodium hydroxide (11.3 mL, 11.3 mmol), and sodium bicarbonate (5.7 g, 67.8 mmol) were added followed by methyl chloroformate (5.24 mL, 67.8 mmol) with vigorous stirring at 0 °C. Stirring was continued at 25 °C for 2 hours. The reaction mixture was concentrated and the residue was dissolved in ethyl acetate (50 mL). The ethyl acetate layer was washed with water (3 x 10 mL) and was dried over anhydrous sodium sulfate. The solvent was removed and the residue was crystallized using ethyl acetate and petroleum ether to obtain the title compound.
Yield: 2.0 g (67 %); $^1$H NMR (CDCl$_3$, 300 MHz): δ 1.43 (t, 3H), 2.63 (s, 3H), 3.80 (s, 3H), 4.39 (q, 2H), 7.68 (d, 1H), 7.78 (s, 1H), 7.95 (d, 1H).

**PHARMACOLOGY**

The efficacy of the present compounds can be determined as described below. The exemplified pharmacological assays have been carried out with the compounds of the present invention and their pharmaceutically accepted salts.

*In vitro* biological experiments:

**Example 10**

**Screening in adipogenesis assay** (A phenotype-based assay)

The assay was designed as in the reference J. Bio. Chem., 278, 7320-24, 2003, which is incorporated herein by reference for disclosure of the assay.

The culture medium was Dulbecco's modified Eagle's medium (DMEM) containing fetal bovine serum to an extent of 10 % by volume. The solution of test compound (20 μg/ml) was prepared in DMSO.

The 3T3 L1 fibroblasts were seeded at $5 \times 10^4$ cells/well in 96-well plates and were incubated at 37 °C in 5% $CO_2$ atmosphere until confluency was reached. The vehicle control contained 1% v/v DMSO and Rosiglitazone at 1 μM was used as standard. TNF (Tumor Necrosis Factor) at 5 ng/ml was used as negative control.

Confluent cells were treated individually with test compound (20 μg/ml), 5 μg/ml insulin was added, and the cells were kept for 3 days in culture medium. The test compound was removed and the cells were maintained for 8 days with replacement of medium every 3 days.

At the end of 8 days, the number of fat filled adipocytes formed in each well was assessed by observing under microscope. The lipid droplets were stained with Oil red O and percentage of stained cells was estimated. Compounds that caused the formation of adipocytes by more than 5 times the vehicle were termed as actives (as illustrated in Figure 1).

Conclusion: Representative compounds of the present invention demonstrated significant adipogenesis.

**Example 11**

**Insulin resistance assay** (IR assay)

The assay was designed as in the reference, British Journal of Pharmacology, 130, 351-58, 2000, which is incorporated herein by reference for disclosure of the assay. The solution of test compound (10 μM/mL) was prepared in DMSO.

Rosiglitazone (0.1 μM in DMSO) was used as standard.

Differentiation into adipocytes was induced by known methods as described below. (J. Biol. Chem., 260, 2646-2652, 1985, which is incorporated herein by reference for disclosure of the induction).

Culture medium containing 0.5 nM 1-methyl-3-isobutylxanthine (IBMX), 0.25 μM dexamethasone, 5 μg/ ml insulin (bovine/human), 10 mM HEPES buffer and fetal bovine serum (FBS) 10 % by volume in Dulbecco's modified Eagle's medium (DMEM) was used for differentiation.

3T3 L1 fibroblasts were seeded in 24-well or 6-well plates at a density of $0.5-2 \times 10^4$ cells/well and were allowed to reach maximal confluency.

The confluent fibroblasts were exposed to culture medium for 2 days. After this period, fresh culture medium (DMEM) containing only insulin was used, 10% FBS was added and cultured for 4 days with change of medium every 2 days. After 7 days the cultures received DMEM containing 10% FBS with no exposure to insulin. By the end of 8-10 days, more than 95% of the cells have become differentiated into adipocytes.

The mature adipocytes were exposed to dexamethasone, 100 nM added in ethanol, in culture medium and incubated for 2 days. On the third day, solution of test compound was added along with 100 nM dexamethasone containing medium for 4 days with a change in medium after every 2 days. Vehicle control contained 1 % v/v of DMSO. Rosiglitazone was used as a standard and was added at a concentration of 0.1 μM in DMSO, along with 100 nM dexamethasone containing medium for 4 days with a change in medium after every 2 days. After a total period of 6 days, the cells were processed for glucose uptake as follows.

The insulin resistant adipocytes were exposed to serum-free DMEM containing 0.1 % bovine serum albumin for 3-4 hours at 37 °C in $CO_2$ atmosphere. The test compound was also present during this period. After 3-4 hours, the medium was aspirated and replaced with Kreb's Ringer phosphate (KRP) buffer at pH 7.4 and with human/ porcine insulin, 200 nM. The cells were incubated for 30 minutes at 37 °C. At the end of 30 minutes, 0.05 or 0.1 μCi of $^{14}$C-2-deoxyglucose was added to each well of either 24-well or 6-well plates respectively and was incubated for exactly 5 minutes. After exactly 5 minutes, the plates were transferred to ice trays and medium was rapidly aspirated. The cell layer was washed twice with ice-cold phosphate buffered saline, (PBS), pH 7.4. Finally the cell layer was lysed with 150 μl of 0.1 % sodium dodecyl sulfate (SDS) and the radioactivity of the cell lysate was determined in liquid scintillation counter. Non specific glucose uptake was assayed in wells exposed to cytochalasin B, an inhibitor of glucose transport. Compounds that showed statistically significant increase in the glucose transport/uptake expressed as CPM/well above the level in cells exposed to insulin vehicle were considered actives in this assay. The cut off limit for activity in this IR assay was defined as the increase 1.5-fold of vehicle, assay value of 1.0 for vehicle. Activity was also expressed as % of Rosiglitazone, which is used as a standard for comparison. Statistical analysis was performed using unpaired t - test.

The results are summarized in Table 1.

Table 1: Activity of compounds in insulin resistance model

| Sr. No. | Compound No. | Fold vehicle* | % of Rosiglitazone** |
|---------|--------------|---------------|----------------------|
| Std | Rosiglitazone | 2.6 ± 0.1 | 100 |
| 01 | 1 | 2.2± 0.18 | 72.5± 2.6 |
| 02 | 2 | 2.62± 0.05 | 105± 3.6 |
| 03 | 3 | 1.48± 0.09 | 31.6± 6.2 |
| 04 | 4 | 1.88± 0.14 | 76.8± 12.4 |
| 05 | 5 | 2.41± 0.06 | 78.7± 3.5 |
| 06 | 6 | 2.10± 0.05 | 61.4± 3.15 |
| 07 | 7 | 1.49± 0.05 | 27.5± 3.0 |
| 08 | 8 | 2.19± 0.09 | 66.4± 5.2 |
| 09 | 9 | 1.01± 0.27 | NIL |
| * fold activity over vehicle<br>** comparison with Rosiglitazone | | | |

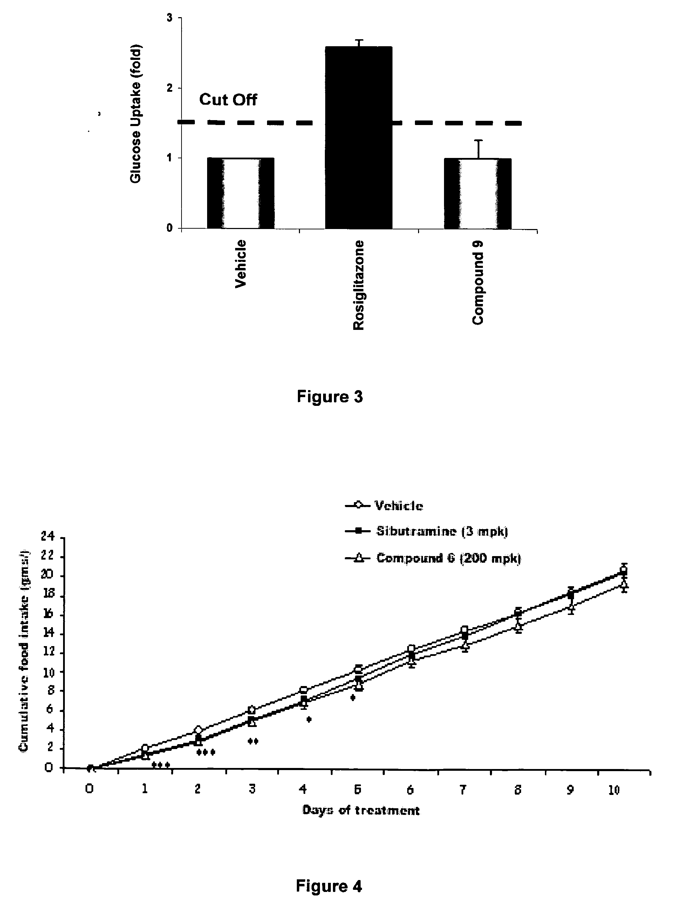

The glucose uptake is illustrated in Figure 2 and Figure 3.

Conclusion: Representative compounds of the present invention showed insulin sensitizing activity in increasing glucose uptake in the insulin resistance model.

### *In vivo* biological experiments:

Note: All animal experimental procedures were approved by Animal Ethics Committee.

The compounds which were found active in example 11 were subjected to *in vivo* evaluation in animal models of insulin resistance.

### Example 12

### Screening in *db*/*db* BU6J mice

The protocol was designed as in references.

1. Metabolism, 53(12), 1532-1537, 2004.
2. American Journal of Hypertension, 17(5), Supplement 1, S32, 2004.

These two references are incorporated herein by reference for disclosure of the protocol.

The screening of compounds was based on their ability to reduce the plasma glucose levels in genetically diabetic *db*/*db* BU6J mice.

Male *db*/*db* mice (obtained from the Animal House of Nicholas Piramal Research Centre, Goregaon, Mumbai, India) were used for this study (body weight in the range of 30-40 g and age is 6-8 weeks) and were kept eight per cage in individually ventilated cages at controlled temperature (22 ±1 °C) and humidity (45 ± 5 %). Food and water were provided *ad libitum* during their laboratory stay, except for four hours fasting prior to blood sample collection. 12 hours light and dark cycle was followed during the whole study period.

After 4 hours fasting blood samples were collected from mice. Mice showing plasma glucose levels between 300 to 500 mg/dl were divided in groups (8-10 per group) such that the mean plasma glucose levels and variation within the group, for each group, is nearly same. After grouping, mice in respective groups received treatment with 0.5% CMC vehicle, standard compound or test compounds for 10 days. Rosiglitazone was used as a standard.

After 4 hours fasting, mice were anaesthetized using isoflurane (inhalation anesthetic), and blood samples were collected through the retro orbital plexus.

Collected blood samples were centrifuged at 7000 rpm for 10 minutes at 4 °C; Separated plasma was used for estimation of plasma glucose using diagnostic kits (Diasys, Germany). Plasma glucose levels of treated groups were normalized with control group using the following formula, which accounted for the changes in control group.

The results are summarized in Table 2.

Table 2: Reduction in the plasma glucose levels in genetically diabetic *db/db* BU6J mice.

| Sr. No | Compounds tested in *db/db* mice | | | Rosiglitazone tested in *db/db* mice | |
|---|---|---|---|---|---|
| | Compound No. | Dose (10 days) | Normalization with control* | Dose (10 days) | Normalization with control* |
| 01 | 1 | 100 mpk bid | 42.93 ±4.95 | 5 mpk bid | 47.25 ±3.94 |
| 02 | 2 | 100 mpk bid | 45.14 ± 4.92 | 5 mpk bid | 45.34 ± 2.74 |
| 03 | 3 | 50 mpk od | Inactive | 10 mpk od | 40.12 ± 12.53 |
| 04 | 4 | 50 mpk bid | 24.99 ± 17.92 | 10 mpk od | 44.62 ± 6.88 |
| 05 | 5 | 150 mpk od | 43.2±15.7 | 10 mpk od | 93.8 ± 22.7 |
| 06 | 6 | 100 mpk bid | 46.07 ± 4.55 | 5 mpk bid | 55.73 ± 4.46 |
| 07 | 7 | 100 mpk bid | Inactive | 5 mpk bid | 53.34 ± 4.11 |
| 08 | 8 | 100 mpk bid | 41.90 ± 3.84 | 5 mpk bid | 54.52 ± 4.92 |

Formula used for normalization:

* : {1- (Ratio of mean plasma glucose levels of control group on day 10 to day 0) / (Ratio of plasma glucose levels of treated group on day 10 to day 0)}x100 Conclusion: Representative compounds of the present invention showed significant glucose lowering activity in the animal model of diabetes.

## Example 13

## Evaluation of antiobesity

## Chronic study

The assay was designed as in the reference, British Journal of Pharmacology, 132, 1898-1904, 2001, the disclosure of which is incorporated by reference for the teaching of the assay.

Male C57BI6/J mice (3-4 weeks of age) were housed in groups of 10 animals per cage in the animal facility. High fat diet (D12451 Research Diets Inc., New Brunswick, NJ 08901,USA, 45% kcal from fat) and water was provided *ad libitum* for 14 weeks. After this period, the animals were housed individually in cages. The animals were weighed and separated into groups with similar body weight. They were acclimatised to the experimental procedures for 2 days. Animals were dosed intraperitoneally with test compound (200 mpk) or the standard (3 mpk) in 10 mL/kg of 0.5%CMC vehicle between 10:00 am -12:00 noon. After drug administration the animals were presented with a pre-weighed amount of food in the food cup. Weight of feed remaining in the cup and body weight were recorded daily just prior to dosing. The change in weight and cumulative food intake were computed.

The results are indicated in Figure 4 and Figure 5.

Conclusion: Compound 6 is effective in reducing cumulative food intake of diet induced obese (DIO) mice during the 10 days of treatment. (*** p< 0.001, ** p < 0.01, * p < 0.05 vs vehicle treated controls)

Compound 6 is effective in reducing cumulative body weight gain of diet induced obese (DIO) mice during the 10 days of treatment. (*** p< 0.001, ** p < 0.01, * p < 0.05 vs vehicle treated controls).

## Example 14

## Evaluation of lipid levels (dyslipidemia)

The assay was designed as in the reference, Metabolism, 49 (1), 22-31, 2000, the disclosure of which is incorporated by reference for the teaching of the assay.

Seven groups of male *db/db* mice (8 animals per group) were used. Animals were orally dosed twice a day (bid) for an extended period of fifteen days, with either the vehicle or compound 5 (5 mpk, 25 mpk, 50 mpk, 100 mpk and 200 mpk) or with the standard drug, Rosiglitazone (5 mpk). Body weight was measured daily. On day 15, the animals were deprived of food for 4 hours after the last dose administration. Blood was collected at the end of the 4-hour period using heparinised capillaries by a retro-orbital puncture. Plasma samples were analyzed for glucose, triglyceride, cholesterol, using the autoanalyser.

Compound 5 exhibited triglyceride-lowering ability in *db/db* mice at all the doses tested. The compound caused plasma triglyceride reductions ranging from 28% to 42%) with the higher doses inducing higher reduction. Rosiglitazone, in the same study caused 40% decrease in plasma triglyceride levels.

Compound 5 tested at doses higher than 50 mpk, induced 26% reduction in cholesterol levels Rosiglitazone lowered cholesterol levels by a similar extent of 27 %.

Conclusion: In *db/db* mice, Compound 5 was as efficacious as Rosiglitazone, in lowering lipid levels.

**Conclusion:**

The IR assay has effectively detected true *in vivo* active compounds as shown in examples 1-8, wherein all compounds which were active in IR assay were also active in *in vivo* model.

The compounds which tested negative in the *in vivo* assay were not positives in the insulin resistance assay thus giving the specificity of 100 %. Moreover the probability of predicting *in vivo* activity based on the insulin resistance assay is 100 % provided compounds have very good absorption and pharmocokinetics parameters. These observations indicate that the insulin resistance assay is superior to other methods in predicting plasma glucose lowering activity in an insulin resistant *in vivo* model such as *db/db* mice.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

All publications and patent applications in this specification are indicative of the level of ordinary skill in the art to which this invention pertains.

The invention has been described with reference to various specific and preferred embodiments and techniques. However, it should be understood that many variations and modifications may be made while remaining within the spirit and scope of the invention.

**Claims**

1.  A compound which is:

    2-{4-[2-(tert-Butoxycarbonyl-methyl-amino)-ethoxy]-benzyl}-2-methoxymalonic acid dimethyl ester;
    3-{4-[2-(tert-Butoxycarbonyl-methyl-amino)-ethoxy]-phenyl}-2-methoxypropionic acid;
    3-{5-[2-(Benzooxazol-2-yl-methyl-amino)-ethylamino]-1-oxo-1,3-dihydroisoindol-2-yl}-propionic acid ethyl ester;
    2,4-Dichloro-N-[5-chloro-6-(isoquinolin-3-yloxy)pyridin-3-yl]-benzenesulfonamide;
    N-{6-[4-(4-Acetyl-piperazin-1-yl)-phenoxy]-5-chloro-pyridin-3-yl}-2,4-dichlorobenzenesulfonamide; or
    N-[5-Chloro-6-(quinolin-3-yloxy)-pyridine-3-yl]-4-methoxy-benzene sulfonamide;

    or a pharmaceutically acceptable salt or solvate or crystalline form thereof, for use in the treatment of disorders caused by insulin resistance to glucose uptake and disorders related thereto.

2.  A compound according to claim 1, wherein the disorders caused by insulin resistance comprise Type 2 diabetes, obesity, glucose intolerance, dyslipidemia, hyperinsulinemia, atherosclerotic disease, polycystic ovary syndrome, coronary artery disease, hypertension, aging, non alcoholic fatty liver disease, infections, cancer and stroke.

3.  A compound according to claims 1 or 2, wherein the disorders caused by insulin resistance to glucose uptake comprise type 2 diabetes and obesity.

4.  A compound according to claim 3, wherein the disorder caused by insulin resistance is type 2 diabetes.

5.  A compound according to claim 3, wherein the disorder caused by insulin resistance is obesity.

6. A compound according to claims 1 or 2, wherein the disorder caused by insulin resistance is dyslipidemia.

7. A method of identifying lead compounds which act as insulin-sensitizers, comprising:

   screening test compounds in a phenotype-based assay; and screening compounds identified as actives in the phenotype-based assay in an insulin resistance assay.

8. A method according to claim 7, wherein the phenotype-based assay is an adipogenesis assay.

9. A method according to claim 8, wherein screening in the adipogenesis assay comprises screening against 3T3-L1 adipocytes.

10. A method according to claim 7, wherein the insulin resistance assay is an *in vitro* model of insulin resistance.

11. A method according to claim 10, wherein the *in vitro* model of insulin resistance comprises dexamethasone treated 3T3-L1 adipocytes.

12. A method of claim 7, further comprising screening the lead compounds for activity in an animal model of insulin resistance, comprising measuring plasma glucose levels in genetically diabetic *db*/*db* BU6J mice.

13. A method of claim 7, further comprising screening the lead compounds for activity in an animal model of insulin resistance comprising measuring food intake and body weight gain in diet-induced obese C57Bl6/J mice.

Figure 1

Figure 2

Figure 3

Figure 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Diabetes Care,* 1997, vol. 15, 318-368
- *Diabetes,* 1988, vol. 37, 1595-1607
- *Diabetes,* 2004, vol. 53, 1633-42
- *Diabetes,* 1992, vol. 41, 598-604
- *J. Clin. Invest.,* 1992, vol. 89, 782-788
- *J. Am. Med. Association,* 2001, vol. 285, 2486-2497
- *Current opinion in Biotechnology,* 2004, vol. 15, 564-571
- *Mutation Research,* 2005, vol. 586, 115-123

- *J. Bio. Chem.,* 2003, vol. 278, 7320-24
- *British Journal of Pharmacology,* 2000, vol. 130, 351-58
- *J. Biol. Chem.,* 1985, vol. 260, 2646-2652
- *Metabolism,* 2004, vol. 53 (12), 1532-1537
- *American Journal of Hypertension,* 2004, vol. 17 (5), 32
- *British Journal of Pharmacology,* 2001, vol. 132, 1898-1904
- *Metabolism,* 2000, vol. 49 (1), 22-31